# EUROPEAN PATENT APPLICATION

(11) **EP 1 738 684 A1**
(43) Date of publication of application: **03.01.2007**
(21) Application number: 06253372.4
(22) Date of filing: 28.06.2006
(51) Int. Cl.: A61B 5/00, A61B 5/103

(54) **Handheld device for determining skin age, proliferation status and photodamage level**

(30) Priority: 29.06.2005 US 170129
(71) Applicant: JOHNSON & JOHNSON CONSUMER COMPANIES, INC., Skillman, NJ 08558 (US)
(72) Inventor: Cole, Curtis A., Ringoes, NJ 08551 (US); Kollias, Nikiforos, Skillman, NJ 08558 (US); Hartman, Frederick, Englishtown, NJ 07726 (US)
(74) Representative: Tunstall, Christopher Stephen

(57) **Abstract**

A self-contained, handheld probe for measuring at least one parameter of skin condition, has one or more light sources that may be used to project light upon the skin. The light projected is of a selected wavelength known to generate a specific fluorescence that is indicative of the skin parameter of interest in accordance with a known correlation. To produce the proper excitation light, a light source generating that wavelength is used or a broader spectrum of light is selectively filtered to pass the wavelength of interest. Lenses, fiber optic elements or waveguides may be employed to project the light onto the skin at a specific location and/or to deliver the skin response to a light detector, which measures the light signal from the skin. and generates an output signal indicative of the value of the at least one parameter. The probe may be used to measure skin age, photodamage and/or proliferation.

## Description

### Field of the Invention

The present invention relates to apparatus and methods for testing the skin, and more particularly, for evaluating characteristics of skin based upon the skin's fluorescence characteristics when illuminated with light of a selected range of wavelengths.

### Background of the Invention

The monitoring and maintenance of healthy skin is an important concern for most people. Typically, people examine their skin using a mirror in a setting with natural, incandescent and/or fluorescent lighting. This self examination process is used by a person to ascertain the condition of their skin and potentially to treat the skin with various therapies and preparations in order to improve the condition of the skin. For example, upon viewing the skin in the mirror and ascertaining that the skin looks oily, the selection and use of a washing and/or drying agent may be employed. The presence of wrinkled skin may indicate that a moisturizer or other wrinkle treatment would be advisable. Beside visual inspection, consumers have little concrete scientific information regarding the status of their skin's health, particularly elements relating to the skin aging processes and the extent of invisible photodamage beneath the skin surface. The first signs of skin "aging" noticed by consumers are fine lines and wrinkles around the eyes, yellowing of the skin, and development of pigmented spots. At this point, the majority of the skin damage has been done, and the process of repair is difficult if not impossible. In addition to the skin conditions that are readily visible in normal lighting environments, there are conditions and indicators of skin health and age that are invisible to inspection using a mirror in typical lighting. For example, subsurface conditions of the skin, such as UV photo damage to subsurface layers (mainly due to exposure to the sun), etc., will not necessarily be apparent by simply viewing the surface of the skin in a mirror. It is now known that inspection of the skin utilizing various wavelengths of light and/or polarized light can illuminate and reveal skin conditions which would otherwise be imperceptible. In addition, these alternative illuminating techniques can highlight and emphasize visible conditions, such as wrinkles or acne. Known techniques for sub-surface or enhanced surface viewing typically involve photography, wherein a flash unit which is capable of producing light of a particular wavelength is activated and an image captured with a camera. Various filters may also be employed in this process. Ultraviolet (UV) photography utilizing a flash unit filtered to produce ultraviolet A light and a camera that is filtered so that only visible light enters the lens produces images that are visually enhanced with regard to pigmentation, the presence of the bacteria p. acnes and horn. A variation of ultraviolet photography has been termed the "sun camera" where ultraviolet A light is used to illuminate the skin and an ultraviolet A sensitive digital camera is used to record the ultraviolet light reflected from the skin. In this arrangement, both pigment distribution and the surface features of the skin are visually enhanced. While the foregoing photographic techniques have proven valuable and useful for analyzing the condition of the skin, they require fairly sophisticated and expensive equipment and the use of photographic techniques and are difficult to quantitate. In addition to photographic techniques, spectrometric apparatus and techniques are also known for evaluating skin condition. One such technique measures fluorescence of the skin in response to light in the 295 nm excitation wavelength range as an indicator of skin age. Prior spectrometric analysis techniques required expensive laboratory instruments and a trained technician to collect and analyze the data gathered. There is a need therefore for an inexpensive and uncomplicated apparatus and method for evaluation and quantitation of the skin's overall health as measured by it's proliferative status, overall physiological "age" and the extent of photodamage of particular skin areas, that would be suitable for consumer use.

### Summary of the Invention

The problems and disadvantages associated with conventional apparatus and techniques utilized to view or assess the skin's condition are overcome by the present invention, which includes a probe for measuring at least one parameter of skin condition, including an illuminator for generating optical radiation to be projected upon the skin to be examined. A detector measures the optical signal from the skin in response to the excitation energy projected on the skin by the illuminator and generates an output signal indicative of the value of the at least one parameter. The probe is a self-contained unit that may be held in a human hand.

### Brief Description of the Drawings

FIG. 1 is a side view of a device in accordance with an embodiment of the present invention for measuring the skin condition;
FIG. 2 is a diagrammatic view of interior components of the invention of FIG. 1, e.g., as revealed by taking the cross-section of FIG. 1;
FIG. 3 is an end view of the invention of FIG. 1;
FIG. 4 is an alternative embodiment of the invention shown in FIG. 3;
FIG. 5 is a diagrammatic view of an embodiment of the present invention;
FIG. 6 is a block diagram of an embodiment of the present invention;
FIG. 7 is a schematic diagram of an embodiment of the present invention;
FIGS. 8 and 9 are graphs showing a correlation between age and skin fluorescence emission at 500 nm wavelength when exited by light of 400nm; and
FIG. 10 is a graph of skin health related to age.

### Detailed Description of the Invention

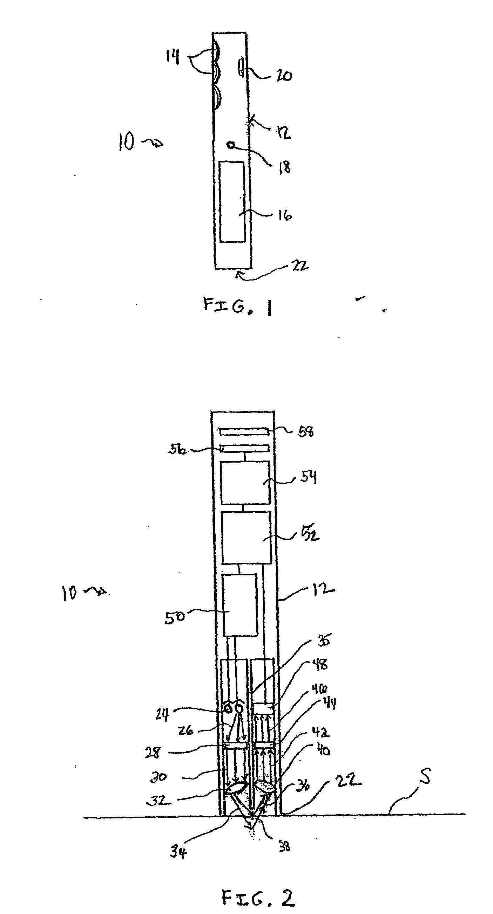

FIG. 1 shows a probe 10 having an elongated housing 12, dimensioned for retention in a single hand of a user. The probe 10 has a plurality of controls 14, e.g., in the form of buttons which may be depressed to allow the user to select a particular test to be conducted and to initiate testing. An LCD (liquid crystal display) 16 is provided on the probe 10 to display instructions and test results to the user. In taking test measurements with the probe 10, a ready light 18 indicates when the testing can be initiated, e.g., certain tests may require that the probe 10 be seated against the skin at the skin contact end 22 to exclude environmental light from passing between the skin contact end 22 and the skin. When referring to radiation, the term "light" as used herein describes optical radiation in the wavelength regions from the ultraviolet region through the infra-red regions and is not confined to the wavelengths that are only detected by the human eye. A zero reading of light sensed would indicate proper seating of the probe, thus triggering illumination of the ready light 18. A USB port 20 is provided on the probe 10 to allow data that is collected by the probe 10 to be downloaded to a computer or a storage device.

FIG. 2 diagrammatically shows the interior components of the probe 10, which include one or more lights or other optical radiation sources 24 for generating light 26 for illuminating the skin S. The output 26 from the light 24 is passed through an illumination filter 28 which may be used to select a particular range or set of wavelengths. The filtered light 30 passes through the filter 28 and impinges on an illumination lens 32 which redirects and/or focuses light 34 upon the skin S at a selected location. Light 36 may be reflected from the skin surface S or may penetrate the skin causing the skin to emit or fluoresce light 38. The reflected and/or emitted light 36, 38 is directed towards the detection lens 40 which focuses and redirects the light signal 42 to a detector filter 44. The detector filter 44 may be utilized to filter out undesired wavelengths of light and pass the selected wavelengths of interest 46. The filtered light 46 from the skin S impinges upon detector 48 which senses the intensity of the light 46 of the selected wavelengths. This intensity measurement is provided to a microprocessor 52 which may include suitable circuitry for converting an analog signal to digital data. The operation of the light or lights 24 is controlled by light controller 50 under the direction of the microprocessor 52. The microprocessor 52 would include a memory for storing the signal data generated by the detector 48. A transceiver 54 acting through antenna 56 may be utilized to communicate the data received from the detector 48 to a remote computer. Alternatively, the transceiver 54 and antenna 56 may be utilized to download instructions from a computer. A battery 58 is provided for powering the above described components of the probe 10.

FIG. 3 shows the skin contact end 22 of the probe 10. A separator 35 terminates prior to contact with the skin S thereby allowing reflected light 36 (see FIG. 2) to be received by the detector lens 40. In the alternative, the separator 35 could extend to the skin surface to be coextensive with the contact end 22, thereby occluding reflected light 36 and allowing only light 38 emitted from below the surface of the skin S to enter the detector lens. In contrast, second and third separator walls 60 and 62 are coterminal with the skin contact end 22 thereby preventing surface reflections from a second illumination aperture 64 from entering the second detector aperture 66. A separator 35 need not be used if the detector filter 44 filters out all but the desired reflected/emitted wavelength(s) and the detection lens 40 is shielded from those desired wavelength(s) present in ambient lighting.

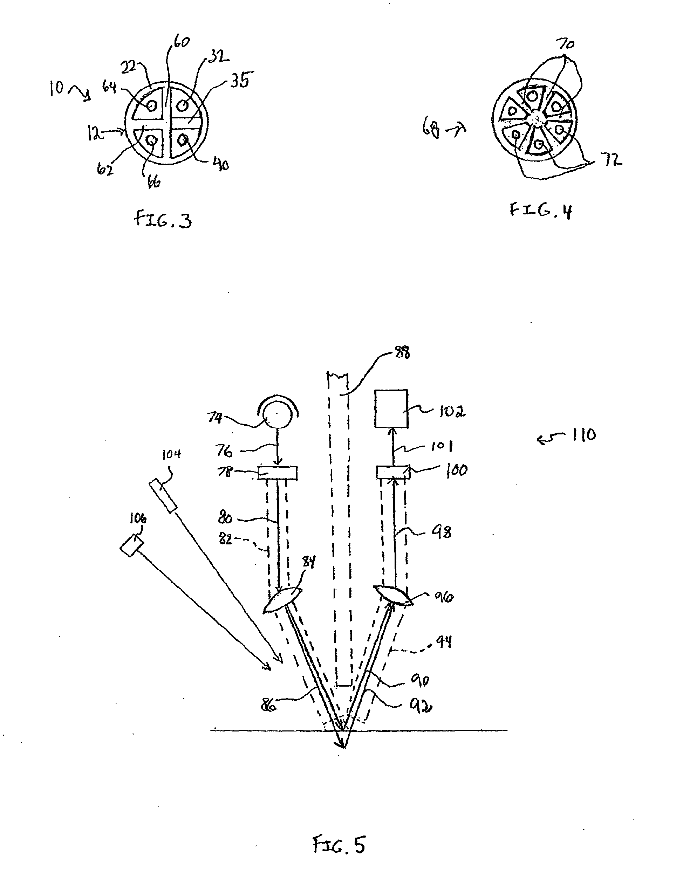

FIG. 4 illustrates another embodiment of the probe 68 wherein a plurality of separators 70, some or all of which allow reflection from the skin's surface into an adjacent detector aperture 72 or alternatively may be coterminal with the probe skin contact end thereby blocking reflected light.

FIG. 5 diagrammatically illustrates the process conducted by the present invention 110 and shows additional alternatives pertaining to illumination and detection. More particularly, to measure the skin proliferation rate, light of 295nm is utilized as the excitation light 86 impinging upon the skin S. The desired wavelength range of the emitted light from the skin monitored by detector 102 is 340nm. Light reflected 90 or fluoresced/emitted 92 from the skin is passed through the lens 96 and through detector filter 100, which eliminates all wavelengths except for those in the 340nm range. The monitored emission 101 is detected by the detector 102, generating a signal to the microprocessor 52. In measuring the skin proliferation rate utilizing 295nm excitation light and measuring the emission of 340nm light from the skin, the light source 74 may be a flash lamp with a 295nm narrow pass filter 78 (plus or minus 10 to 20nm) or a fluorescent bulb coated with a specific phosphor that emits within this range with little or no emission at 340nm. The output of the fluorescent lamp can also be filtered with a narrow band pass filter 78 to make the source more monochromatic (295nm plus or minus 10 to 15nm). The light source 74 could also be a mercury lamp without a phosphor coating on the bulb envelope which is filtered through a narrow band pass filter 78 to isolate the 296.7nm wavelength excitation. As an alternative to the light source 74 and filter 78 combination, a xenon -chloride laser source 104 could be used to excite the skin, in which case the laser 104 would generate illuminating/excitation light of 308nm. It should be observed that if a laser is utilized, the lens 84 is not necessarily required unless the physical layout of the probe 110 requires the laser beam to be spread or to redirected to the desired focal point on the surface of the skin. The detector 102 for measuring skin proliferation rate (295nm excitation/ 340nm emission) may be a silicon-based semiconductor photocell filtered with a narrow band pass filter 100 (to limit the radiation reaching the photo cell to wavelengths between 335 to 350nm with high blockage of radiation below 335nm). Alternatively, a long pass Schott filter such as a WG335 filter of 3mm thickness could also be used to block the short wavelengths. As a further alternative, ordinary window glass of about 2 mm thickness could be used as the filter 100 to block the short UVB emission wavelengths.

FIG. 5 shows that a fiber optic 82 could be utilized to transmit light 80, 86 to the skin on the illumination/ excitation side of the probe 110. Similarly, a fiber optic 94 could be utilized for receiving the reflected light 90 and/or emitted light 92 from the skin on the detector side of the probe 110. In either instance, the lenses 84, 96 may or may not be utilized depending upon the physical layout of the probe 110, e.g., depending upon whether the fiber optic elements 84, 94 are adequate to position the excitation light 86 on the proper focal point of the skin, and the receiving fiber optic 94 is positioned correctly to absorb the admitted radiation 90, 92 for detection. Still referring to FIG. 5, when the probe 110 is utilized to determine chronological skin age, light in the range of 400 nm is used for excitation/ illumination and light of 500nm is monitored on the detector 102 side of the probe 110. In that particular application, the light source 74 may be an LED 106 with emitting wavelengths between approximately 380nm and 420nm. Alternatively, the light source 74 may be a flash lamp, such as a xenon arc lamp filtered with a narrow band pass filter 78, which allows passage of wavelengths between 380nm to 420nm. Alternatively, the light source 74 may be a fluorescent light with emissions in the 380 to 420nm wavelength region, with or without a narrow band pass filter 78. As yet a further alternative, a mercury vapor lamp could be utilized as the light source 74 which emits wavelengths in the 400 to 410nm range that are filtered with along band pass filter 78 such as a UV400 cut-off filter to limit exposure to UV radiation. As yet a further alternative, the light source 74 may be a tungsten-halogen light source that admits a continuous spectrum of wavelengths that are filtered with a narrow band pass filter 78 permitting the passage of light in the 380 to 420nm region. When measuring chronologic skin age, the monitoring photo detector 102 may be a silicon photocell with a filter 100 to block out wavelengths below 470nm. Any other type of semiconductor photocell that produces a signal based on the photoelectric effect of light to measure light intensity may be used. A long pass filter blocking wavelengths below 470nm could also be utilized with such a photocell.

FIG. 6 shows a block diagram of an embodiment of the probe system 120 including a power control system 122 which would distribute power to the circuit components of the probe 120 from the power system 123, for example a battery or batteries, in a conventional manner. The power control system provides suitable voltages to the various components of the system, i.e., the illumination system 124, the light control system 126, the detection system 128, etc. Preferably, the power control system 122 includes a timer that causes automatic shutdown to conserve battery power if there is a lack of activity over a predefined time period. The power system 123 may be a replaceable battery and/or a recharging system for recharging rechargeable batteries via an external charger which could be plugged into the probe 120. As described above, the illumination system 124 may include one or a plurality of different light sources, which may emanate a full spectrum of light or may provide light in a narrower wavelength range, e.g., an LED. The illumination system 124 preferably has a predetermined maximum capacity for illumination. The light control system 126, as described above, may include lenses, fiber optics or waveguides to direct, transform and funnel the light emanating from the illumination system 124 to and from the skin. As noted above, filters within the light control system 124 may be utilized to block specific wavelengths and to pass particular wavelengths of light on the way to the surface of the skin (excitation) and/or returning from the surface of the skin for detection. Preferably, the housing is provided with a means to prevent undesired ambient light from interfering with the detection system 128 for certain tests. As noted above, the detection system includes a photodetector for sensing light intensity. Data concerning the intensity of light received by the detection system 128 is conveyed to the main control system 130, which may include a digital processor. The probe 120 may be analog or may include a digital processor. The main system control 130 responds to operator input to initiate readings and otherwise controls the other components of the probe 120 to coordinate their activities. The main system control 130 presents the results obtained by operating the probe 120 to the operator via an LCD screen, or by remote communication through a transceiver to a computer. This is shown as the operator interface system 132 which may be in the form of an LCD, a transceiver, and/or a communications link e.g., USB to an external computer. A USB connector may be utilized to charge the batteries 58 of the device 10, as well as to exchange data. The operator interface system 132 may display the value of the skin fluorescence to the operator and may also display system status, errors and operator directions.

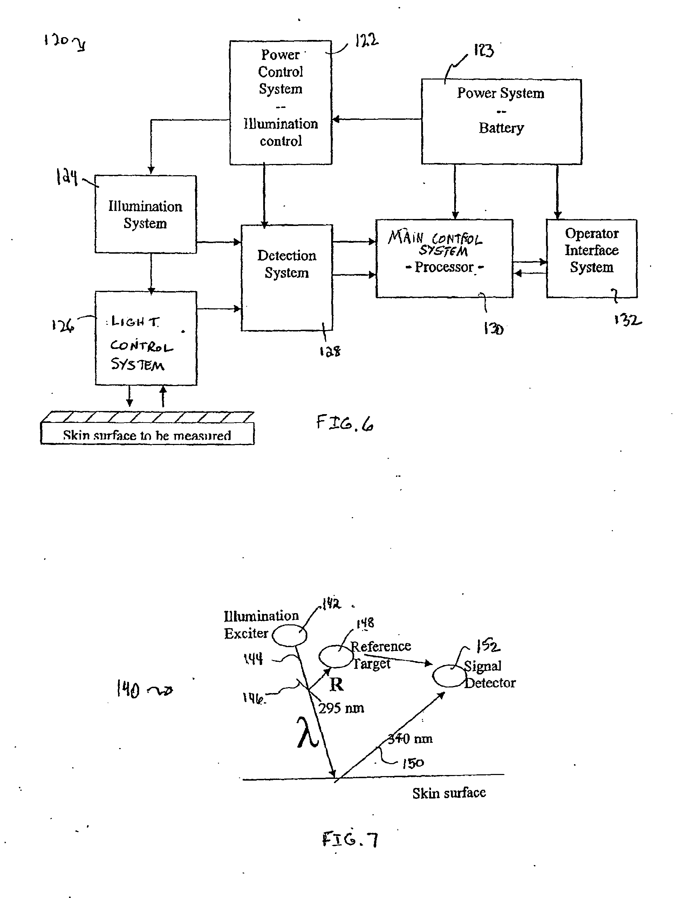

FIG. 7 shows an embodiment of the probe system 140 utilizing an illumination source/exciter 142, which generates light 144 for impinging upon the surface of the skin and for penetrating the surface to cause sub-surface fluorescence. The illumination light 144 produced by the exciter 142 may be 295nm plus or minus 10nm. In the UV-B band, maximum exposure to the skin should not exceed 10 mJ/cm² to prevent erythogemic responses from the skin. As before, a signal detector 152 measures the amount of light 150 emanating from the skin and is blind to the illumination light 144 and ambient light. The amount of light detected by the detector 152 may be displayed to the operator/user as a value from 0 to 1,000 for example. The probe system 140 may include a reflector 146 and a reference target 148. The reflector 146 redirects the illuminating light 144 onto a signal detector 148- the reference target, which may be the same signal detector 152 for the purpose of establishing a reference value from the illuminating light 144. In operation, the operator powers the probe system 140, whereupon the probe system checks itself and auto-calibrates itself. The operator then places the unit over the skin to be tested and presses a "read" button. The illumination light 142 illuminates the skin via the optical system, be that via wave guides, optical fibers or lenses, as is required. The reference value of the illumination light 144 is read at the reference target 148 by virtue of the operation of the reflector 146 which directs the illumination light 144 at the reference target 148. The light is then redirected back to the skin causing fluorescence, e.g., producing a 340nm responsive emission from the skin. The signal detector 152 reads the fluorescence level as light signal 150 and converts the ratio of the fluorescence level over the reference level to a displayed value of 0 to 1,000 indicating the proliferation level. For example, a ratio of zero may have an output of zero and ratio of 1 to 1 may correspond to an output of 1,000. The operator may then read the fluorescence level from the LCD display of the probe system 140. Upon release of the "read" button, the illumination light 142 is powered off and the operator interface system 132 will maintain the reading until the unit powers down or is shut off. Subsequent pressing of the "read" button may overwrite previous readings.

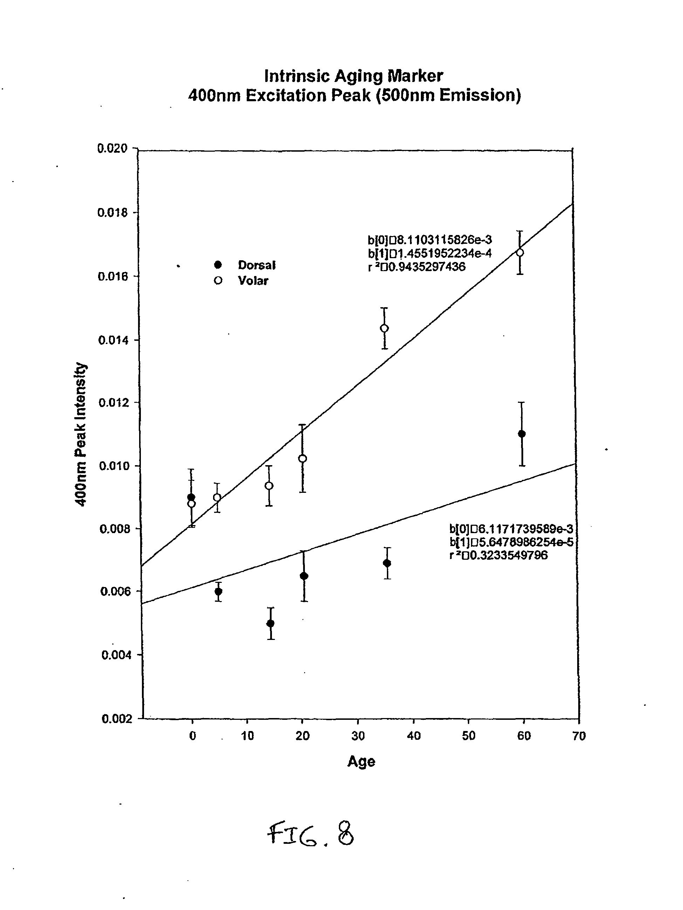

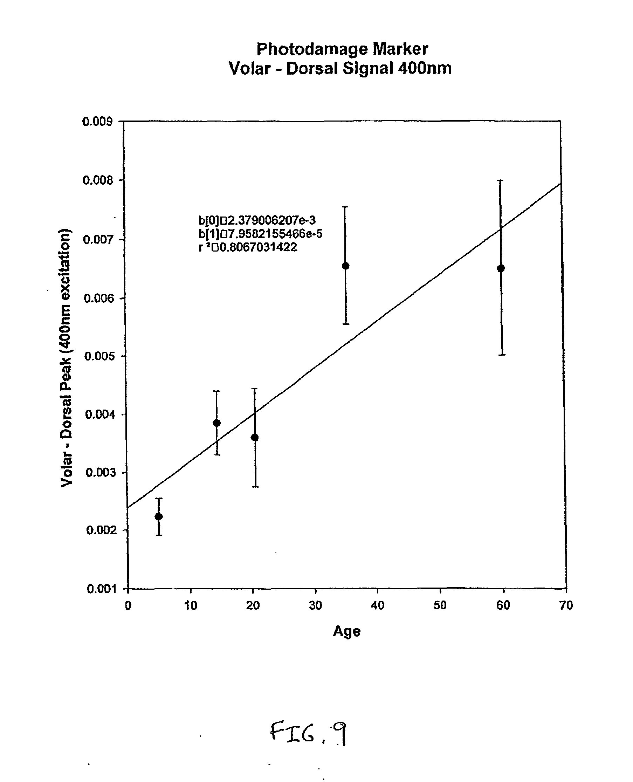

FIGS. 8 and 9 are graphs showing the correlation between skin fluorescence at 500nm to skin photodamage/age. The determination of the extent of photodamage is conducted by measuring the 400nm excitation/500nm fluorescence emission on two skin sites, i.e., one that is routinely exposed to sun - such as the forearm or the face (dorsal), and then obtaining a second measurement at a site that is typically non-exposed ― such as the upper inner arm, or on a non-exposed buttock or upper thigh area (volar). The difference in fluorescence between dorsal and volar surfaces has been shown to correlate with photodamaged skin age and chronological age.

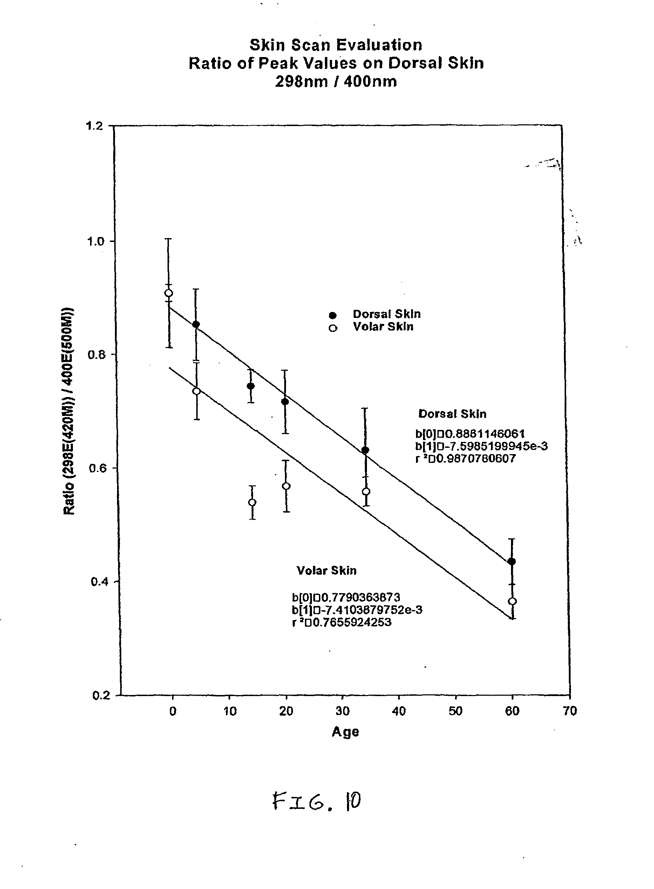

FIG. 10 is a graph showing the correlation between the ratio of fluorescence behavior over two emission bands and age (skin health). As indicated in U.S. Patent Application No. 10/735,188, entitled Method of Assessing the Skin by Kollias and Stamatas, filed December 12, 2003 (attorney docket no. J&J 5092), which is incorporated by reference herein, the fluorescence ratio of the 295nm excitation: 340nm emission/390 excitation: 480 emission signals is a measure of skin health that is highly correlated with age. These measures can be conducted on photodamaged or non-photodamaged skin and yield results that are highly correlated with skin age. These measurements are conducted with the same instrumentation and techniques described above, with electronic circuitry doing the computations to determine the ratio and correlate the results versus age, as presented by Kollias and Stamatas in the referenced application. Accordingly, the probe 10 of the present invention can be utilized to measure skin age and the degree of photodamage.

It is understood that while the invention has been described in conjunction with the detailed description thereof, that the foregoing description is intended to illustrate and not limit the scope of the invention, which is defined by the scope of the appended claims. Other aspects, advantages, and modifications are within the scope of the claims.

## Claims

1. A probe for measuring at least one parameter of skin condition, comprising:
an optical radiation source for generating light to be projected upon the skin to be examined;
an optical radiation detector for measuring the light signal from the skin in response to the light projected on the skin by the optical radiation source and
generating an output signal indicative of the value of the at least one parameter, said probe being a self-contained unit that may be held in a human hand.

2. The probe of Claim 1, wherein said optical radiation source includes a light source and transmission means, said transmission means controlling the light generated by the optical radiation source and delivering it to the skin and said optical radiation detector, said optical radiation detector including a photodetector and detector transmission means, said detector transmission means controlling light from the skin and delivering it to the photodetector.

3. The probe of Claim 2, wherein said probe is capable of measuring a plurality of parameters indicative of skin condition.

4. The probe of Claim 3, wherein said probe has a plurality of different optical radiation sources, such that the light generated by a first differs from the light generated by a second.

5. The probe of Claim 3, wherein said probe has a plurality of different optical radiation detectors, such that an output signal from a first differs from an output signal from a second.

6. The probe of Claim 3, wherein said probe can measure at least one of proliferation, photodamage and age.

7. The probe of Claim 2, wherein the at least one parameter is skin proliferation.

8. The probe of Claim 7, wherein the light generated by the optical radiation source includes wavelengths in the range of approximately 295 nm and the wavelength range of the light corresponding to the generation of the output signal from said optical radiation detector is approximately 340 nm.

9. The probe of Claim 2, wherein the at least one parameter is photo damage.

10. The probe of Claim 9, wherein the wavelength of the light generated by the optical radiation source includes wavelengths in the range of approximately 400 nm and the wavelength range of the light corresponding to the generation of the output signal from said optical radiation detector is approximately 500 nm.

11. The probe of Claim 2, wherein the at least one parameter is skin age.

12. The probe of Claim 7, wherein the wavelength of the light generated by the optical radiation source includes wavelengths in the range of approximately 380 to 420nm and the wavelength range of the light corresponding to the generation of the output signal from said optical radiation detector is approximately 480 nm to 520 nm, respectively.

13. The probe of Claim 7, wherein the optical radiation source includes at least one of a flash lamp in combination with a narrow pass filter, a fluorescent bulb coated with a specific phosphor that emits in the range of approximately 295 nm, a fluorescent lamp filtered with a narrow pass filter, a mercury lamp filtered by a narrow pass filter, a Light Emitting diode (LED), and a xenon-chloride laser.

14. The probe of Claim 7, wherein the optical radiation detector includes at least one of a photocell filtered by a narrow pass filter, a long pass Schott filter and window glass.

15. The probe of Claim 11, wherein the optical radiation source includes at least one of an LED, a flash lamp filtered with a narrow band pass filter, a fluorescent light, a mercury vapor lamp filtered with a long pass filter, a tungsten-halogen light filtered by a narrow pass filter.

16. The probe of Claim 15, wherein the optical radiation detector includes at least one of a photocell in combination with a long pass filter.

17. The probe of Claim 2, wherein said transmission means and said detector transmission means is at least one of a lens, a fiber optic, and a waveguide.

18. The probe of Claim 2, wherein said optical radiation source and said optical radiation detector are positioned beside each other with a separator therebetween.

19. The probe of Claim 1, wherein the light signal from the skin includes reflected light.

20. The probe of Claim 1, wherein the light signal from the skin includes fluorescent emissions.

21. A method of determining skin proliferation status using the probe of Claim 7, wherein skin fluorescence is measured at about 340nm when the skin is illuminated with light at approximately 295nm.

22. A method of determining skin age using the probe of Claim 11, wherein skin fluorescence is measured at about 500nm when the skin is illuminated with light at approximately 400nm.

23. A method of determining skin photodamage by measuring skin age by the method of Claim 22 on undamaged skin and subtracting the value of the skin age on a UV exposed site.
